# EUROPEAN PATENT SPECIFICATION

(11) **EP 2 235 498 B1**
(45) Date of publication and mention of the grant of the patent: **13.02.2013**
(21) Application number: 07857636.0
(22) Date of filing: 14.12.2007
(51) Int. Cl.: G01N 3/08, G01M 11/08

(54) **METHOD OF EVALUATION OF OPTICAL FIBERS DURING MANUFACTURING THEREOF**
VERFAHREN ZUR BEWERTUNG OPTISCHER FASERN WÄHREND DEREN HERSTELLUNG
PROCÉDÉ D'ÉVALUATION DE FIBRES OPTIQUES PENDANT LEUR FABRICATION

(43) Date of publication of application: 06.10.2010
(73) Proprietor: Prysmian S.p.A., 20126 Milano (IT)
(72) Inventor: SCAFURO, Nicola, I-20126 Milano (IT); VITOLO, Matteo, I-20126 Milano (IT); TESTA, Gerardo, I-20126 Milano (IT); MAZZOTTI, Andrea, I-20126 Milano (IT); RUSSO, Paolo, I-20126 Milano (IT)
(74) Representative: Maccalli, Marco
(86) International application number: PCT/EP2007/064002
(87) International publication number: WO 2009/076998

(56) References cited:
- EP-A- 0 393 878
- WO-A-2006/058551
- JP-A- 56 128 437
- US-A- 4 148 218
- US-A- 4 501 492
- US-A- 4 601 208
- US-A- 5 076 104
- US-A- 5 410 396
- US-A- 5 647 884
- US-A- 5 842 373
- US-A1- 2002 069 675
- US-B2- 6 892 589

## Description

### Background of the invention

### Field of the invention

The present invention generally relates to optical fibers and to the manufacturing thereof. In particular, the present invention relates to a method for the evaluation of optical fibers during their production, particularly during fiber drawing from a preform.

### Description of the related art

Optical fibers, particularly those intended for telecommunications applications, need to exhibit good optical properties. These properties include, for example, low attenuation and low pulse spread.

Another important property that optical fibers should exhibit is a sufficiently high tensile strength. Tensile strength is a mechanical property which an optical fiber needs to possess (at least to a given extent) in order to ensure that damages and breakages of the optical fiber do not occur during installation thereof.

In order to protect the optical fiber surface against mechanical and chemical damages, the optical fibers are usually coated, during the drawing process from a fiber preform, by applying a synthetic coating thereto.

However, even coated fibers frequently exhibit a low tensile strength, for example because particles in the oven used to heat the preform, or other foreign particles, damages the fiber surface while the fiber is still in the oven chamber (for example, impurities present in the oven chamber may cause defects on the fiber surface which reduce the fiber strength), or due to important variations in the drawing process working conditions (*e.g*., modifications of the cooling conditions or problems occurring in the fiber coating application step). Also, other impurities that are already present in the preform from which the fiber was drawn can be the cause of fiber defects that worsen the fiber tensile strength characteristics. The tensile strength of the fiber depend in fact on the frequency and the size of these defects, which in general are statistically distributed over the entire fiber length.

Therefore, the tensile strength of an optical fiber needs to be assessed to ensure that a sufficiently high tensile strength value is guaranteed during installation of the optical cable comprising the optical fiber.

In the past, the testing of the fiber tensile strength was directly done on-line, typically downstream from the optical fiber drawing step (particularly just before the winding of the drawn optical fiber onto the collecting reel). The whole drawn fiber length was tested on-line by applying (using a suitable apparatus) to the fiber being drawn a tensile force adapted to induce a strain that mimics the stress conditions that the fiber will undergo when placed in an optical cable, in particular during the installation thereof. The strain value is usually specified in optical fibers' data sheets, and is for example equal to 1%, although the specific fiber strain that an optical fiber is subjected to on the field may depend on the specific application; for example, for submarine optical cables installation, the strain may be up to 2%. This on-line screening was performed on the whole drawn fiber length because it performed also the quality check which is always carried out on the finished product (*i.e*., no further checks were envisaged on the final product, off-line of the manufacturing process). The on-line screening is disclosed, for instance, in U.S. patent No. 4,148,218, U.S. patent No. 6,892,589 and U.S. patent No. 4,601,208.

The presence of defects causes breakages of the fiber being drawn, which are easily detected. However, many breakages negatively affect the fiber draw rate, and make the drawing process quite discontinuous. Another problem resides in that it has been found that the fiber immediately after drawing is somewhat more resistant than after a certain time has passed. Therefore, the on-line screening of the fiber immediately after drawing (by inducing a 1% strain) does not actually remove all the defects.

More recently, in optical fiber manufacturing plants the fibers are subjected to a so-called "off-line screen test" or "off-line screening". The drawn fiber is unwound from the after-drawing collecting reel, conducted over rollers, and loaded with a predetermined tensile force over its entire length (suitable to induce the fiber strain specified in the fiber data sheet, for example 700 MPa for a 1% strain); the applied force causes the fiber to break in correspondence of the defects, and most of them can thus be eliminated. Typical values of the applied force are such as to cause a 1% strain on the fiber. Thanks to the high degree of purity guaranteed by the modern fiber manufacturing plants, typical average distances between two consecutive defects in the fiber are larger than various tens of kilometers. The off-line screen test is disclosed, for instance, in U.S. patent No. 5,076,104.

However, the off-line fiber screening step has the main drawback that the possible poor quality of the starting preform (*e.g*., due to the presence of foreign particles within the preform or on the preform outer surface, or due to variations occurred to the working conditions during the formation of the preform because of the complex and delicate drawing step of the optical fiber due, for instance, to the very low diameters, very high speed rates, very high vertical towers), from which the optical fiber has been drawn, can be detected only when the tensile strength testing step is performed onto the manufactured optical fiber which has been obtained from that preform. The poor quality of the starting preform results in a high number of consecutive breaks which occur on the drawn optical fiber during the tensile strength testing step thereof. Since a significant lowering of the average lengths between two consecutive breaks (sometimes even lower than the minimum commercial span of an optical fiber, usually in the order of about 5 km) is not acceptable, the manufactured optical fiber has to be discarded. This clearly represents a very high cost for the manufacturer who has wasted expensive raw materials (especially with respect to the optical fiber coating) as well as time without obtaining any result.

Moreover, in case the poor quality of the preform is due to a problem of the manufacturing process, since the testing step is carried out off-line (*i.e*. when the optical fiber has already been manufactured) and thus some time after the optical fiber has been obtained, the negative results of the tensile test can be possibly acknowledged when more than one preform has already been produced and drawn, so that a great amount of optical fiber has to be discarded.

In order to test the preforms from which optical fibers are to be drawn, the preforms could be optically examined. Examples of these methods are disclosed in the article by H. M. Presby et al, "Optical Fiber Preform Diagnostics", published in "Applied Optics", Vol. 18, No. 1, Jan. 1, 1979, pp. 23-30. A method of non-destructively testing optical specimens has also been disclosed in the U.S. patent No. 4,501,492.

However, these optical methods applied on the preforms, before the drawing process, do not take into account the fiber defects that originate during the preform drawing process. According to the cited U.S. patent No. 4,501,492, it should even be possible to analyze the fiber during the process, but it is certainly hard to have a good spatial resolution at high speed rate.

Accordingly, an optical inspection of the preform cannot reveal the defects which may originate during the successive drawing step for obtaining the optical fiber. Moreover, in case the optical inspection is carried out also on the optical fiber while being drawn from the preform, this method is far from being effective to detect possible defects in the drawn optical fiber since a good spatial resolution cannot be ensured at the high speed rates which are used in the modern optical fiber manufacturing processes.

In EP-A-0393878 an optical fiber is proof tested by bending it relative to the fiber axis by a preselected amount in all directions and at all locations along the length of the fiber. In one approach, the optical fiber is wrapped around a cylindrical mandrel in a helical pattern and drawn over the mandrel through its entire length. With the preferred helical angle of about 45 degrees, two wraps of the optical fiber around the mandrel ensure that all of the fiber will be bent in all directions. If a flaw exists in the optical fiber that would cause failure at any amount of bending below that of the preselected amount, this proof test will cause the fiber to fall so that the weak point may be eliminated.

US 2002/069675 discloses a method and apparatus for automatic threading and winding of optical fiber onto various components in a fiber draw system, as well as methods and apparatus for conducting online tensile screening of optical fiber at high speeds. In a preferred embodiment, the fiber is tensile tested during fiber draw and wound directly onto a shipping spool to be shipped to a customer. The tensile stress can be imparted to the fiber during the draw process by feeding the fiber through a screener capstan, which works in conjunction with another capstan to impart the desired tensile stress to the fiber during the draw process.

US 4601208 discloses an optical fiber proof testing equipment having a rotary drive and two pulleys directly coupled to the drive. A fiber is taken from fiber drawing equipment around part of the circumference of the first pulley, along a guide track and around part of the circumference of the second pulley. Respective continuous belts apply a resilient bias to clamp the fiber to the pulleys, the resilient bias being adjustable. The second pulley is marginally greater in diameter than the first pulley so as to establish a predetermined tensile stress in fiber moving between the two pulleys. The test equipment is used after fiber has been drawn in a drawing tower and then coated and before the fiber is stored on reels. The tensile force on the fiber can be chanced by changing the diameter of the first or second pulleys. In addition some incremental change in the tensile force can be obtained by adjusting the resilient bias provided by the continuous belt.

US 4,418,218 discloses an apparatus for applying tensile stress to an optival fiber.

### Summary of the invention

The Applicant has tackled the problem of devising an optical fiber evaluation method that is not, or at least less, affected by the problems of the known fiber screening methods mentioned above as far as the fiber tensile strength is concerned. In particular, the Applicant has faced the problem of checking the quality of an optical fiber during drawing thereof, thereby avoiding that defective optical fibers are manufactured (which would be unsuitable for being commercialized and thus which would have to be discarded) and that raw materials, power energy, man time and machine time are wasted.

The waste of time and material is even greater since, usually, the optical fiber is discovered to be defective during the off-line screen test when the obtained optical fiber is subjected to the prescribed tensile stress for checking the quality thereof.

The Applicant has perceived that the use of preforms of good quality (*i.e*., substantially devoid of defects), as well as the fact that the prescribed values for the process parameters are correctly used during the manufacturing process of the optical fiber can be guaranteed by preliminarily testing the tensile strength of a very first length of an obtained optical fiber at a very first stage of the drawing thereof, said testing being carried out on-line during the optical fiber manufacturing process.

In particular, the Applicant has perceived that by applying a suitable tensile stress (which is lower than the tensile stress used in the off-line fiber screening for checking the quality of the produced optical fiber) only to an initial length of a drawn optical fiber during the drawing thereof the quality and productivity of the optical fiber manufacturing process is advantageously increased. Typically, said initial length depends on the statistical distance between fiber breakages; preferably, said initial length is of approximately 10 Km. Therefore, if the optical fiber passes the test, it can be assumed that also the remaining optical fiber to be produced from that preform (typically some hundreds of kilometers of drawn fiber are obtained from a preform) will pass the test and substantially no discards (*i.e*. defective optical fibers which have a high and unacceptable number of consecutive breaks) will be obtained from such preform. Moreover, the Applicant has also perceived that, if the optical fiber passes the preliminary tensile strength test (*i.e*. by carrying out an on-line testing of said initial length of drawn optical fiber), the quality of the drawing process can be considered to be satisfactory and not to introduce any further defects in the drawn optical fiber during the drawing of the preform.

The Applicant has found that the technical problem mentioned above can be solved by providing the optical fiber manufacturing process with a tensile strength testing step according to which a predetermined tension is applied to the very first length of the drawn optical fiber, said tension being thereafter removed during the drawing of the remaining optical fiber length which is obtained from the preform.

Thanks to the present invention, preforms having poor mechanical strength, as well as anomalies in the process working conditions, will be detected during the preliminary tensile strength testing step since the tension applied to the fiber being drawn causes a break in the optical fiber substantially at every defect in the optical fiber and due to defective preforms and/or drawing process anomalies. Therefore, by checking the tensile strength of the optical fiber at a very initial stage of the optical fiber manufacturing process, it is possible to evaluate the preform quality and the process working conditions so that the resulting optical fiber is expected to possess the desired tensile strength value, thereby reducing the process discards.

According to the present invention, a method of evaluation of an optical fiber during manufacturing thereof is provided, as defined in claim 1.

The method may further comprise submitting the drawn optical fiber to an off-line tensile strength test by subjecting the whole drawn optical fiber to a predetermined second tensile stress.

After said stopping the fiber drawing process, the following steps may be performed:
- cleaning the fiber drawing tower;
- restarting the fiber drawing process;
- subjecting a predetermined further length of the optical fiber while being drawn to the predetermined first tensile stress;
- if the detected number of fiber breakages exceeds a predetermined second threshold, stopping again the fiber drawing process.

The method may further comprise, after said stopping again the fiber drawing process, discarding the fiber preform.

Said first tensile stress is in particular lower than said second tensile stress.

In particular, said first tensile stress is lower than or equal to 60% of said second tensile stress.

Said first tensile stress may be between 20% and 50% of said second tensile stress.

Said first tensile stress may be selected in the range from approximately 200 g to approximately 1000 g, more preferably from approximately 300 g and approximately 600 g.

Said predetermined initial length may be at least about 10 Km.

Said predetermined first threshold may be equal to 2 or 3.

Said predetermined second threshold may be equal to 0.

### Brief description of the drawings

These and other features and advantages of the present invention will be made clear by the following detailed description of an embodiment thereof, provided merely by way of non-limitative example, to be read in conjunction with the attached drawings, wherein:
**Figure 1** schematically shows an optical fiber drawing apparatus;
**Figure 2** schematically shows a device according to an embodiment of the present invention, adapted to be used in the optical fiber drawing apparatus of **Figure 1****,** for applying a tensile strength to an optical fiber being drawn;
**Figures 3A** and **3B** schematically show the device of **Figure 2** in two different operating conditions;
**Figure 4** is a schematic flowchart of a method according to an embodiment of the present invention for on-line fiber screening; and
**Figures 5A, 5B** and **5C** are diagrams reporting probabilities of fiber breakages observed in three different sample lengths of drawn optical fiber, obtained by two drawing processes.

### Detailed description of an embodiment of the invention

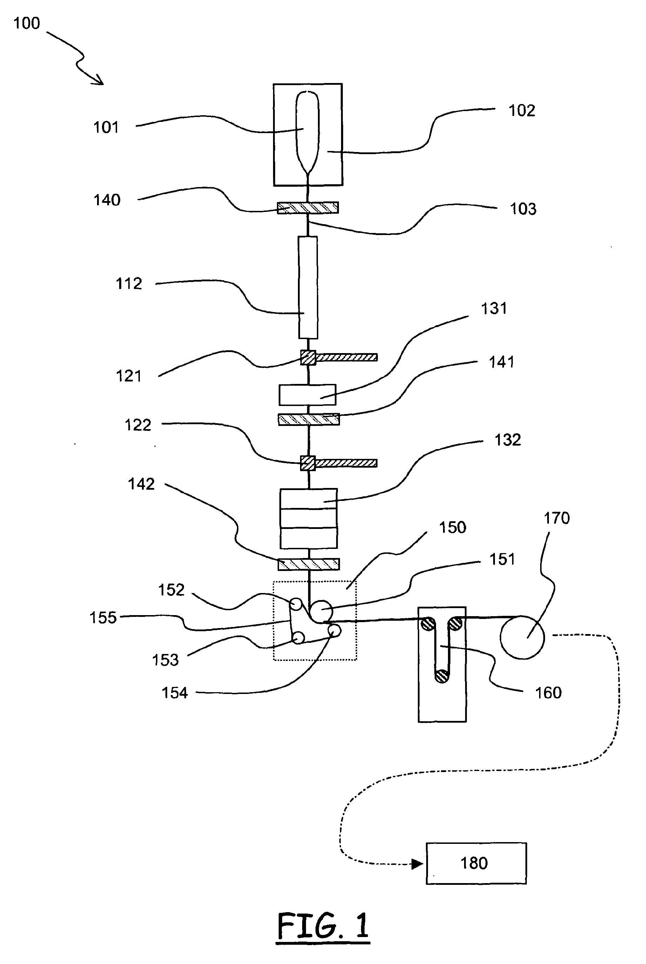

With reference to the drawings, in **Figure 1** an apparatus, globally denoted **100**, for drawing optical fibers is schematically shown. The apparatus **100** is typically inserted in a fiber drawing tower (not shown in the drawing).

A glass preform **101** is drawn into a furnace (oven) **102** to obtain an optical glass fiber **103**. The fiber **103** is pulled down by a tractor apparatus **150**. The diameter of the fiber **103** is measured by a first diameter measuring device **140**. In case of deviation from a target value (e.g., 125 µm) the first diameter measuring device **140** is adapted to send a signal to the tractor apparatus **150** which will vary its rotational speed appropriately.

The fiber **103** is cooled down when passing through a cooling tube **112**, down to a temperature typically lower than 50°C, and the fiber **103** then passes through a first applicator device **121,** adapted to apply onto the fiber **103** a first layer of coating material, typically in the form of a viscous resin.

Then, the fiber **103** passes through a curing device **131,** comprising one or more UV (UltraViolet) lamps, which causes the full or at least partial curing (crosslinking) of the resin forming the first coating layer (the degree of curing depending on the UV power and/or fiber drawing speed).

The diameter of the fiber **103** covered with the first coating layer is measured by a second diameter measuring device **141**. The measured value can be used as a feedback to control the proper working conditions of the cooling tube **112**, in order to adjust the temperature at which the fiber **103** enters into the dyes of the first applicator device **121** (the amount of resin which the fiber can drag, that is, the thickness of the first coating layer, depends on the dye geometry and on the temperature of the incoming fiber with respect to that of the resin).

The fiber **103** then passes through a second applicator device **122**, which applies a second layer of coating material, for example again in the form of a viscous resin. Then, the fiber **103** passes through a second curing device **132**, comprising one or more UV lamps, which causes the full or at least partial curing of the resin forming the second coating layer (again, the degree of curing depends on the UV power and/or fiber draw speed).

The diameter of the fiber **103** covered with the two coating layers is then measured by a third diameter measuring device **142**.

In the tractor apparatus **150**, the fiber **103** is pressed by a belt **155** and partially wrapped on a roller **151**. The belt **155** passes over three rollers **152**, **153** and **154**, so as to have a prescribed tension and assure the friction necessary to pull the fiber **103** downwards.

According to an embodiment of the present invention, after the tractor device **150**, a fiber tensioning device **160** is inserted in-line in the fiber drawing apparatus **100** in the drawing tower, and it is adapted to submit the fiber **103** to an extra-tension during the fiber drawing. The fiber tensioning device **160** will be described in detail later on.

After the fiber tensioning device **160**, a fiber winding device, known per-se, causes the fiber **103** to be wound on a bobbin **170**.

Block **180** schematically denotes a conventional off-line fiber screen test apparatus, adapted to submit the whole drawn fiber to a predetermined tensile stress.

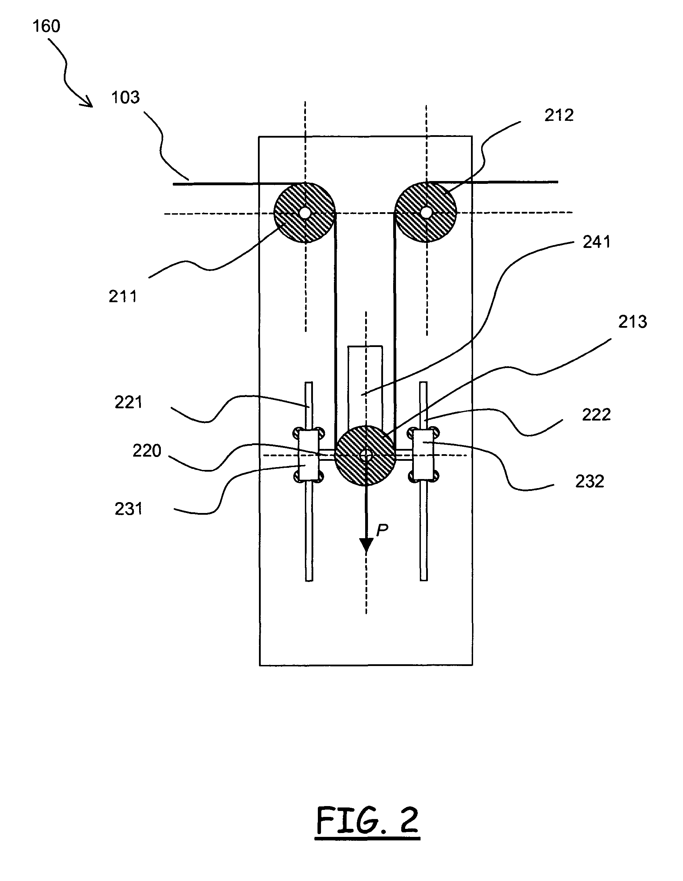

An embodiment according to the present invention of the fiber tensioning device **160** is shown in detail in **Figure 2**. It is pointed out that the embodiment shown in **Figure 2** is not limitative to the present invention, and the extra-tension can be applied to the fiber in other ways, using different fiber tensioning devices.

The fiber tensioning device **160** comprises three pulleys or rollers **211, 212** and **213**. The roller **213** is located in the middle between the rollers **211** and **212**, and it is mounted on a bar **220**, connected to two carriages, **231** and **232**, which can freely move along two vertical guides **221** and **222**. The bar **220** can be loaded with an additional, variable weight **241**. The overall weight of the roller **213**, the bar **220**, the carriages **231** and **232**, and the additional weight **241** will be referred to as the "overall weight" hereinafter, and it is indicated with ***P*** in the drawing.

In operation, the optical fiber **103**, while being drawn and after having been coated, passes through the three rollers **211, 212** and **213**. Due to the overall weight ***P***, the fiber **103** is subjected to a tension equal to ***P***/2. The value of the overall weight ***P*** applied to the fiber univocally corresponds to a certain fiber strain, which further depends on the Young modulus *E* of the optical fiber and on the diameter thereof; optical fibers are normally made of silica, having a Young modulus *E* of about 72 GPa, and have a typical diameter *D* = 125 ± 1 µm, thus an overall weight ***P***, corresponding to an applied tension *T* = ***P***/2, causes a strain equal to *T* divided by the fiber cross section, divided by the Young modulus *E*: *T*/((3.14 * (*D*/2)²) * ***E*).**

The weight **241** can be removed and/or varied during the fiber drawing process.

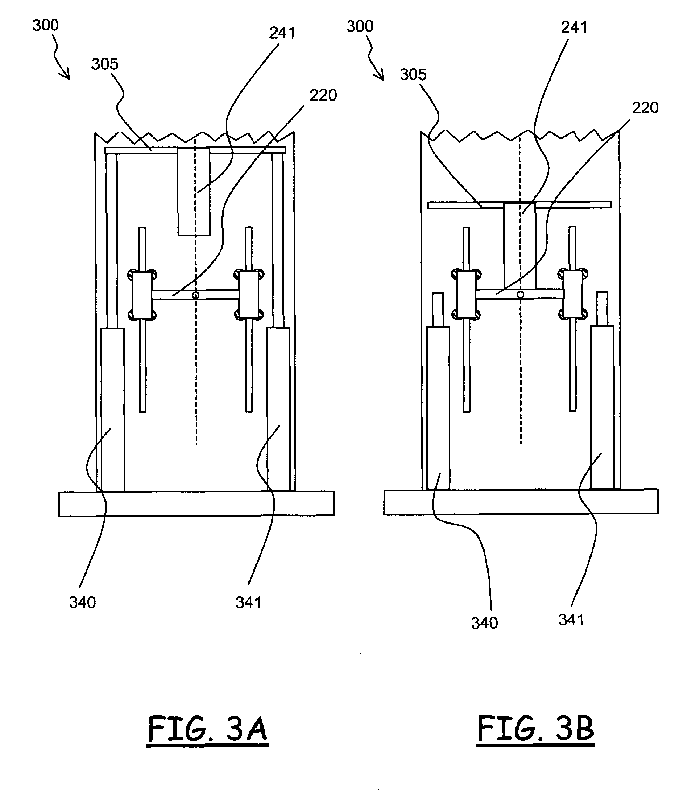

In **Figures 3A** and **3B** a device **300** is schematically shown that is adapted to apply or remove the additional weight **241** onto the bar **220** on which the central roller **213** is mounted (the roller **213** is not depicted in **Figures 3A** and 3B, for the sake of clarity).

In particular, in **Figure 3A** the device **300** is shown in a first (deactivated) working condition, in which the additional weight **241** is not loaded on the bar **220**, being sustained by two, e.g. pneumatic, plungers **340** and 341, through a thin bar **305**. In this condition, the overall weight ***P*** corresponds to the overall weight of the roller **213**, the bar **220**, and the carriages **231** and 232, and it is relatively low, such that a substantially negligible strain is induced on the fiber due to this weight.

In **Figure 3B** the device **300** is shown in a second (activated) working condition, in which the weight **241** is loaded on the bar **220,** since the two pneumatic plungers, **340** and **341**, are collapsed and do not sustain the thin bar **305** carrying the additional weight **241**. The value of the additional weight **241** is such that a prescribed strain is induced on the fiber.

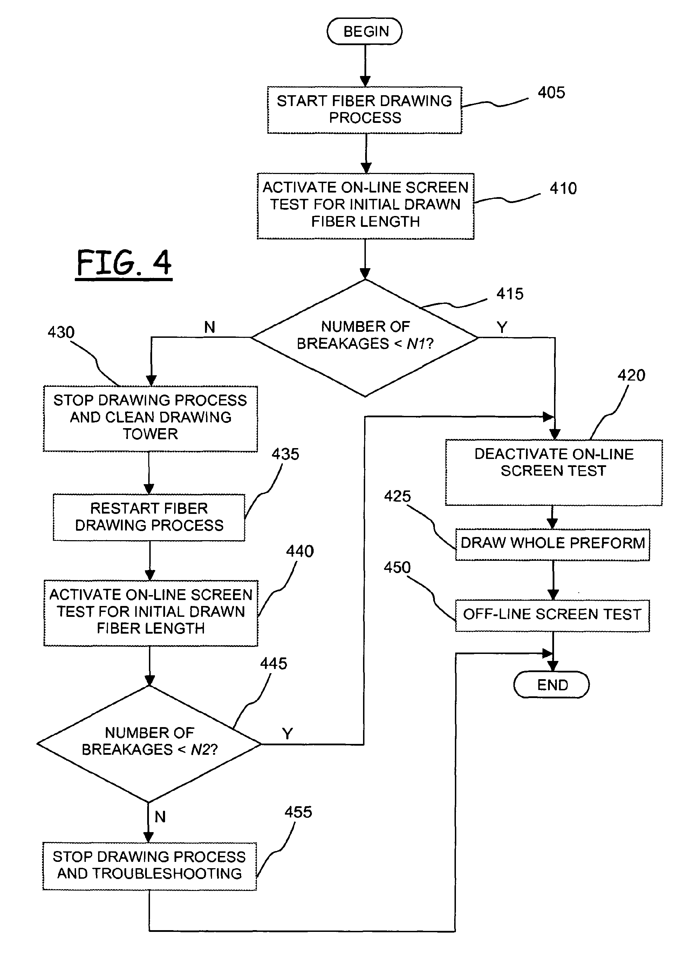

A fiber evaluation method according to an embodiment of the present invention, for the evaluation of fiber tensile strength properties during the fiber drawing, can be the following (reference is made to the schematic flowchart of **Figure 4**).

At the start of the fiber drawing process (block **405**), the on-line fiber screening is activated (block **410**). The device **300** for applying the additional weight **241** is activated (condition shown in **Figure 3B****),** so that the additional weight **241** is applied to the fiber **103** being drawn. The application of the additional weight **241** causes an extra-tension to be applied to the fiber, and this extra-tension induces a prescribed strain in the fiber **103**.

If the number of fiber breakages occurring in a predetermined, initial length of the total length of fiber obtainable from the preform is less than a predetermined number *N1*, considered critical (decision block **415**, exit branch Y), the on-line fiber screening is deactivated (block **420**): the device **300** is deactivated and put into the condition shown in **Figure 3A**, and the remaining of the preform is drawn conventionally, without applying to the fiber the extra-tension (block **425**).

If, in the predetermined initial length of drawn fiber, the number of fiber breakages occurring during the on-line screening is equal or higher than the predetermined number *N1* (for example one, two or more breakages occur) (decision block **415**, exit branch **N),** the drawing process is paused, and the preform **101** is removed from the hot zone of the furnace **102**. The drawing tower is carefully cleaned and controlled (block **430**). The drawing process is then restarted by operating the same preform **101** (block **435**).

Preferably, the on-line fiber screening is kept activated (*i.e*., the device **300** is maintained in the activated condition, shown in **Figure 3B**) after the drawing process is restarted (block **440**), in order to test a further length of drawn fiber. Possibly said further length is equal to the initial length of drawn fiber tested in the previous step.

If no further fiber breakages occur after said further predetermined initial length of drawn fiber, or, more generally, the number of observed fiber breakages is less than a second predetermined number *N2* (decision block **445**, exit branch **Y**), the on-line fiber screening is stopped (the device **300** is deactivated, as shown in **Figure 3A**), and the remaining of the preform is drawn conventionally (blocks **420** and **425**).

The drawn fiber is then subjected to a conventional off-line screening (block **450**), for example applying to the whole length of drawn fiber a tensile force adapted to induce a 1% strain. To this purpose, the drawn fiber is unwound from the collecting bobbin **170** and it is conducted over rollers and loaded with a predetermined tensile force over its entire length (suitable to induce the fiber strain specified in the fiber data sheet, for example 700 MPa for a 1% strain). The off-line screening is used as the quality check of the drawn fiber. By the off-line screening, possible fiber defects are eliminated.

If instead one or more fiber breakages, more generally a number of fiber breakages equal to or higher than the second predetermined number *N2* occurs in the further length of drawn fiber after the drawing process is restarted (decision block **445**, exit branch **N**), the drawing process is definitely stopped, and a troubleshooting procedure may be performed aimed at trying to discriminate whether the fiber breakages are originated by the furnace, or by the defectiveness of the preform, or by other causes (block **455**); the troubleshooting procedure may correspond to that conventionally performed in case, during the known off-line fiber screening methods, fiber breakages occur.

The extra-tension applied to the fiber being drawn during the on-line fiber screen test, *i.e*., the overall weight ***P*** when the additional weight **241** is applied (*i.e*., the overall weight of the roller **213**, the bar **220,** the carriages **231** and **232**, and the additional weight **241** with the thin bar **305**), should be sufficiently low so as to avoid that too many optical fiber breakages occur and, at the same time, sufficiently high to identify the quality of the obtained optical fiber from the mechanical point of view (tensile strength). The overall weight value ***P*** can be varied by varying the weight of the additional weight **241**.

Preferably, the extra-tension applied to the drawn optical fiber during the preliminary on-line screening is at least 50% of the tension usually applied to the fiber in the off-line screen test. For example, a tension of 1000 g applied to the fiber approximately corresponds to a 1% strain in the fiber, that mimics the stress conditions that the fiber will undergo when placed in an optical cable, in particular during the installation thereof. Preferably, the tension applied to the drawn optical fiber at the on-line screen test is of about 350 MPa.

As resulted from experimental trials conducted by the Applicant, a suitable overall weight **P** may be 800 g, that means that a tension of approximately 800 g/2 = 400 g is applied to the fiber; preferably, the overall weight **P** ranges from approximately 200 g to approximately 2000 g.

The overall weight **P** when the additional weight **241** is not applied (*i.e*., the overall weight of the roller **213,** the bar **220,** and the carriages **231** and **232**) can for example be less than 200 g so that the tension applied to the fiber during the drawing process when the on-line screen test is deactivated is less than 200 g/2 =100 g.

The numbers *N1* and *N2*, and their sum *N* = *N1* + *N2*, set thresholds on the number of breakages that the initial length of the drawn optical fiber can undergo during the preliminary on-line screen test for being considered acceptable.

The predetermined initial length of the drawn fiber can be, for example, about 10 km. Preferably, the predetermined initial length of drawn optical fiber is from about 8 km to about 40 km. More preferably, the predetermined initial length of drawn optical fiber is from about 10 km to 30 km. More generally, the predetermined initial length of the drawn fiber can be chosen as a trade off between the desire of avoiding that an unnecessarily long fiber portion is overstressed, and the desire of testing a sufficiently long fiber section to detect possible defects. The value of the initial fiber length might be determined based on an analysis of the statistical distribution of the distances between successive fiber breakages. For example, a fiber drawing process may be characterized by a Poisson statistical distribution of fiber breakages caused by impurities in the oven or in the preform such that, when the fiber is subjected to an off-line screen test inducing a 1% fiber strain (whereas the average distance between breakages decreases if the strain induced in the fiber increases), the average distance between two consecutive breakages is relatively high, *e.g*. higher than 40 Km, possibly up to approximately 100 Km (this values have been reported in the literature, for example in G.S. Glaesmann, "Optical fiber failures probability predictions from long-length strength distributions", published in International wire & cable symposium proceedings 1991, pages 819-825). It is thus possible to predict the probability of having two or three independent fiber breakage events in a certain fiber length: for example, the probability of having one breakage in 10 Km of drawn fiber is approximately 20%, the probability of having two breakages is approximately 2%, and the probability of having three breakages is approximately 0.2%; thus, choosing an initial length of drawn fiber to be subjected to the on-line screen test equal to 10 Km, and setting the threshold N equal to 3 is reasonable, because the probability of having three fiber breakages within 10 Km of drawn fiber is rather low. By increasing the initial length of drawn fiber which is subjected to the on-line screen test, the breakages probabilities increases; for example, considering 30 Km of drawn fiber, the probability of having three breakages is approximately 30%, thus the threshold *N* of the number of fiber breakages should be increased, for example to 4 or 5. Fiber breakages originating from other causes, like bad drawing apparatus setting, insufficient cleaning of the drawing tower, other problems in the preform, may have a lower average distance, *e.g*. 5 Km.

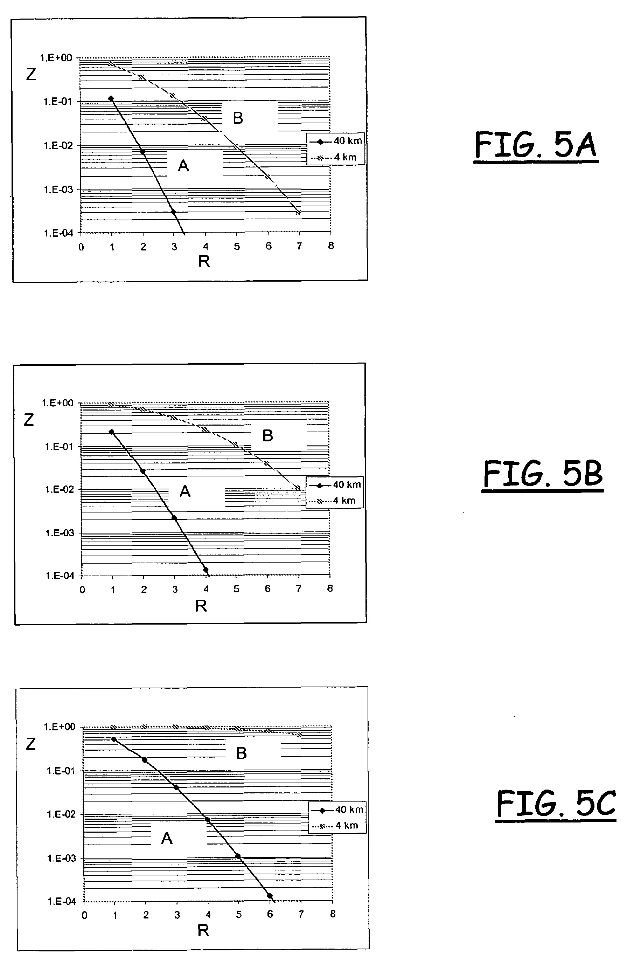

The diagrams in **Figures 5A**, **5B** and **5C** show the probabilities **Z** (in ordinate) of having a certain number of fiber breakages **R** (in abscissa) for two possible fiber drawing processes characterized by: 1) an average distance between breakages of approximately 40 Km when the fiber undergoes a 1% strain (a good process, curves labeled **A**) and 2) an average distance between breakages of approximately 4 Km when the fiber undergoes a 0.5% strain (a bad process, curves labeled **B**), considering drawn fiber spans of 5 Km (**Figure 5A**), 10 Km (**Figure 5B**) and 30 Km (**Figure 5C**). It can be noticed that for fiber lengths of 10 Km (**Figure 5B**), the probability of having three breakages in the bad process is relatively high (approximately 50%), so that a number of three breakages is adapted to discriminate the good fiber drawing process (curve **A**) from the bad process (curve **B**), because the breakage probabilities differ of at least two orders of magnitude). If the considered fiber length is longer, *e.g*. 30 Km (**Figure 5C**), a number of four breakages is adapted to discriminate the good fiber drawing process (curve **A**) from the bad process (curve **B**) because the breakage probabilities differ of at least two orders of magnitude. On the contrary, considering a shorter fiber length, e.g. 5 Km (**Figure 5A**), a number of three fiber breakages would still allow discriminating the good process from the bad process, but the probability of observing fiber breakages in a sample of 5 Km length would decrease in the bad process at values less than 15%, thus resulting useless to detect bad processes.

The Applicant performed the method described above as an alternative to the known off-line fiber screening methods. In particular, one preform has been drawn with the on-line fiber screening activated in the first 10 Km of drawn fiber, as described above, and subsequently another preform has been drawn keeping the on-line fiber screening always deactivated. The result was that on a sample of more than 200,000 Km of drawn fiber, the fiber length lost for breakages occurred during the known off-line fiber screening, that is those fiber portions dose to a breakage or between two consecutive breaks and shorter than the minimal commercial lengths, reduced of approximately 30% in the case the on-line fiber screening was activated compared to the case the on-line fiber screening was deactivated (standard process). The average length between two breaks in the off-line screening has been increased by 25%.

These results turn out in a significant reduction in the fiber production costs.

Several changes to the described method can be envisaged. For instance, the extra tension applied to the fiber being drawn could be between 200 g and 1000 g, more preferably between 300 g and 600 g. The length of fiber screened on line with the apparatus **300** activated vary between 1 km and the whole length of fiber drawn from the preform, more preferably between 5 km and 15 km.

Typically, the tension applied to the produced (drawn) optical fiber at the off-line screen test is of about 1000 g. Preferably, the tension applied to the initial length of optical fiber (during drawing thereof) according to the present invention is lower than or equal to 60% of the tension applied to the drawn optical fiber at the off-line screen test. More preferably, the tension applied to the initial length of optical fiber (during drawing thereof) according to the present invention is between 20% and 50% of the tension applied to the drawn optical fiber at the off-line screen test.

The number *N* of acceptable fiber breakages in the initial length of drawn fiber, *i.e*. the number of breakages threshold, can range from 0 to 5, preferably from 1 to 5. More generally, this threshold is correlated to the length of the drawn optical fiber submitted to the extra tension. Preferably, the number of breakage threshold - when testing a very first length of the drawn optical fiber of about 10 km - for deciding if the on-line screen test has given a positive or negative result is 3. Therefore, in case the number of breaks in said very first length of the drawn optical fiber is ≥ 3, the preform is discarded. This may mean that the drawing process of the fiber is stopped when 2 breaks occur during the drawing of the initial fiber length, then, after the preform is removed from the tower so that the tower can be cleaned and the process parameters can be checked, the drawing process is restarted with the on-line fiber screening activated for testing another length of drawn fiber, the drawing process is definitively stopped, and the preform discarded, in case one further break occurs, so that a total number of 3 breaks (2 breaks during the fist drawing and 1 break at the restart of the process during the second drawing) have occurred in the initial length of the drawn optical fiber.

The method described above causes the fiber being drawn to be submitted to two different stress regimes, depending on the position along the drawn fiber. In fact, the first predetermined length of drawn fiber (*e.g*., an initial 5% of the drawn fiber overall length) undergoes an extra stress corresponding for example to approximately 0.4% of the strain the fiber experiences on the field. If the on-line screening test is passed, the whole preform is drawn with the on-line fiber screening deactivated. If the fiber tensioning device **160** is arranged on-line in the drawing tower just before a conventional winding device, the first predetermined length of the drawn fiber will be wound on the bobbin **170** at a tension equal to half the overall weight ***P***, for example 400 g. This tension will be released after rewinding the fiber on the shipping reels. However, the permanence of the fiber in tension for some time (*e.g*. few hours) could be of concern. The stress aging of the glass fiber is a minor concern, since after drawing the whole fiber is submitted to an off line screening at e.g. 1% strain, and its quality is assured anyway. Moreover, according to accepted fiber lifetime models (COST 218, Optical Materials Reliability and Testing 1791 (1992) 190), a fiber screening performed by submitting the same to a strain of *e.g*. 1% is equivalent to a permanence of the fiber for nearly 10,000 h under a 0.4% stress level.

Some criticality could arise from the viscoelastic behavior of the fiber coating. In fact, the fiber coating layers, when the fiber is submitted to a tension of 400 g on a bobbin, are significantly pressed, and it could take time to completely restore the original configuration. In the worst case, the stress could even produce delamination of the fiber coating. For this reason, the Applicant compared the performances of fiber spans coming from the first predetermined length of drawn fiber, to those of the remaining drawn fiber adopting the IEC Test 60793-1-53 "water immersion". The fiber lengths were immersed in water at 60°C for 30 days, and, at the end, their attenuation at 1550 nm was measured. No significant changes were detected on the 1550 nm attenuation using conventional coating materials, such as those disclosed for example in the U.S. patent No. 7,085,466.

The method according to the present invention is capable of efficiently prevent bad drawing processes. A bad drawing process is one having a significant amount (*e.g*., more than approximately 30%) of drawn fiber lengths which continuously break when submitted to the off-line fiber screening at, for example, 1% strain. Preforms that originate several breakages in the off-line fiber screening at, for example, 1% strain also originate breakages during the on-line fiber screening according to the present invention (even when submitted to a strain that is less than 1%) at the very beginning of the drawing process. This fact makes the proposed method effective in reducing wastes.

The present invention has been here described making reference to an embodiment thereof. Several modifications to the described embodiment, as well as other embodiments are possible, all falling within the scope of protection defined by the appended claims.

For example, the on-line fiber tensioning device used to apply an extra tension onto the initial drawn fiber length could be different from the embodiment described, *e.g*. it could comprise a different number of rollers, and different mechanisms for selectively applying the extra weight ***P*** could be used.

## Claims

1. A method of evaluation of an optical fiber (**103**) during manufacturing thereof, said method comprising:
- subjecting (**410**) a predetermined initial length of the optical fiber to a predetermined first tensile stress while the optical fiber is being drawn from a fiber preform (**101**) ; and
- detecting (**415**) a number of fiber breakages occurring in said initial length of optical fiber:
**characterized in that**:
- said predetermined initial length of the optical fiber is substantially lower than an optical fiber total length, and
- said first tensile stress is lower than the tensile stress used in an off-line tensile strength test for checking the quality of the produced optical fiber,
and **in that** the method comprises:
- if the detected number of fiber breakages exceeds a predetermined first threshold, stopping the fiber drawing process (**430**);
- otherwise removing the first tensile stress from the optical fiber and continuing the drawing process until completion (**420,425**).

2. The method of claim 1, further comprising:
- submitting the drawn optical fiber to an off-line tensile strength test (**450**) by subjecting the whole drawn optical fiber to a predetermined second tensile stress, wherein said first tensile stress is lower than said second tensile stress.

3. The method of claim 1 or 2, further comprising, after said stopping the fiber drawing process:
- cleaning the fiber drawing tower (**430**);
- restarting the fiber drawing process (**435**);
- subjecting a predetermined further length of the optical fiber while being drawn to the predetermined first tensile stress (**440**);
- if the detected number of fiber breakages exceeds a predetermined second threshold (**445**), stopping again the fiber drawing process (**455**).

4. The method of claim 3, further comprising, after said stopping again the fiber drawing process, discarding the fiber preform.

5. The method of claim 2 or of claim 3 or 4 as depending on claim 2, wherein said first tensile stress is lower than or equal to 60% of said second tensile stress, more preferably said first tensile stress is between 20% and 50% of said second tensile stress.

6. The method of any one of the preceding claims, wherein said first tensile stress is selected in the range from approximately 200 g to approximately 1000 g, more preferably from approximately 300 g and approximately 600 g.

7. The method of any one of the preceding claims, wherein said predetermined initial length is at least about 10 Km.

8. The method of claim 7, wherein said predetermined first threshold is equal to 2 or 3.

9. The method of claim 8 as depending on claim 3, wherein said predetermined second threshold is equal to 0.

## Patentansprüche

1. Verfahren zum Bewerten einer optischen Faser (103) während der Herstellung derselben, wobei das Verfahren umfasst:
- Ausüben (410) einer vorgegebenen ersten Zugspannung auf einen vorgegebenen Anfangsabschnitt der optischen Faser, während die optische Faser aus einem Faser-Vorformling (101) gezogen wird; und
- Erfassen (415) einer Anzahl von Faserbrüchen, die in dem Anfangsabschnitt der optischen Faser auftreten;
**dadurch gekennzeichnet, dass**
der vorgegebene Anfangsabschnitt der optischen Faser im Wesentlichen kleiner ist als eine Gesamtlänge der optischen Faser, und
die erste Zugspannung geringer ist als die Zugspannung, die in einem Off-Line-Zugfestigkeitstest zum Prüfen der Qualität der hergestellten optischen Faser eingesetzt wird,
und dass das Verfahren umfasst:
- Unterbrechen des Faser-Ziehprozesses (430), wenn die erfasste Anzahl von Faserbrüchen einen vorgegebenen ersten Schwellenwert übersteigt;
- ansonsten Aufheben der ersten Zugspannung an der optischen Faser und Fortsetzen des Ziehprozesses bis zum Abschluss (420, 425).

2. Verfahren nach Anspruch 1, das des Weiteren umfasst:
- Durchführen eines Off-Line-Zugfestigkeitstests (450) an der gezogenen optischen Faser mittels Ausüben einer vorgegebenen zweiten Zugspannung auf die gesamte gezogene optische Faser, wobei die erste Zugspannung geringer ist als die zweite Zugspannung.

3. Verfahren nach Anspruch 1 oder 2, das des Weiteren nach Unterbrechen des Faser-Ziehprozesses umfasst:
- Reinigen des Faser-Ziehturms (430);
- erneutes Ingangsetzen des Faser-Ziehprozesses (435);
- Ausüben der vorgegebenen ersten Zugspannung (440) auf einen vorgegebenen weiteren Abschnitt der optischen Faser, während sie gezogen wird;
- wenn die erfasste Anzahl von Faserbrüchen einen vorgegebenen zweiten Schwellenwert (445) übersteigt, erneutes Unterbrechen des Faser-Ziehprozesses (455).

4. Verfahren nach Anspruch 3, das des Weiteren umfasst, dass nach dem erneuten Unterbrechen des Faser-Ziehprozesses der Faser-Vorformling verworfen wird.

5. Verfahren nach Anspruch 2 oder 3 oder 4, wenn abhängig von Anspruch 2, wobei die erste Zugspannung 60 % oder weniger der zweiten Zugspannung beträgt und noch besser die erste Zugspannung zwischen 20 % und 50 % der zweiten Zugspannung beträgt.

6. Verfahren nach einem der vorangehenden Ansprüche, wobei die erste Zugspannung in dem Bereich von ungefähr 200 g bis ungefähr 1000 g, noch besser von ungefähr 300 g bis ungefähr 600 g ausgewählt wird.

7. Verfahren nach einem der vorangehenden Ansprüche, wobei der vorgegebene Anfangsabschnitt wenigstens ungefähr 10 km lang ist.

8. Verfahren nach Anspruch 7, wobei der vorgegebene erste Schwellenwert 2 oder 3 beträgt.

9. Verfahren nach Anspruch 8, wenn abhängig von Anspruch 3, wobei der vorgegebene zweite Schwellenwert 0 beträgt.

## Revendications

1. Procédé d'évaluation d'une fibre optique (103) durant sa fabrication, ledit procédé comprenant :
- l'exposition (410) d'une longueur initiale prédéterminée de la fibre optique à une première contrainte de traction prédéterminée alors que la fibre optique est en cours d'étirage à partir d'une préforme de fibre (101) ; et
- la détection (415) d'un nombre de ruptures de fibre survenant dans ladite longueur initiale de fibre optique ;
**caractérisé en ce que** :
- ladite longueur initiale prédéterminée de la fibre optique est sensiblement inférieure à une longueur totale de fibre optique, et
- ladite première contrainte de traction est inférieure à la contrainte de traction utilisée dans un essai de résistance à la traction hors ligne pour contrôler la qualité de la fibre optique produite,
et **en ce que** le procédé comprend :
- si le nombre détecté de ruptures de fibre dépasse un premier seuil prédéterminé, l'arrêt du processus d'étirage de fibre (430) ;
- autrement, la suppression de la première contrainte de traction de la fibre optique et la poursuite du processus d'étirage jusqu'à la fin (420, 425).

2. Procédé selon la revendication 1, comprenant en outre :
- l'exposition de la fibre optique étirée à un essai de résistance à la traction hors ligne (450) en exposant la fibre optique étirée complète à une deuxième contrainte de traction prédéterminée, dans lequel ladite première contrainte de traction est inférieure à ladite deuxième contrainte de traction.

3. Procédé selon la revendication 1 ou 2, comprenant en outre, après ledit arrêt du processus d'étirage de fibre (430) :
- le nettoyage de la tour d'étirage de fibre (430) ;
- le redémarrage du processus d'étirage de fibre (435) ;
- l'exposition d'une autre longueur prédéterminée de la fibre optique, au cours de l'étirage, à la première contrainte de traction prédéterminée (440) ;
- si le nombre détecté de ruptures de fibre dépasse un deuxième seuil prédéterminé (445), l'arrêt à nouveau du processus d'étirage de fibre (455).

4. Procédé selon la revendication 3, comprenant en outre, après l'arrêt à nouveau du processus d'étirage de fibre, la mise au rebut de la préforme de fibre.

5. Procédé selon la revendication 2 ou la revendication 3 ou 4 lorsqu'elle dépend de la revendication 2, dans lequel ladite première contrainte de traction est inférieure ou égale à 60 % de ladite deuxième contrainte de traction, de préférence ladite première contrainte de traction est comprise entre 20 % et 50 % de ladite deuxième contrainte de traction.

6. Procédé selon l'une quelconque des revendications précédentes, dans lequel ladite première contrainte de traction est sélectionnée dans la plage d'environ 200 g à environ 1000 g, de préférence d'environ 300 g à environ 600 g.

7. Procédé selon l'une quelconque des revendications précédentes, dans lequel ladite longueur initiale prédéterminée est d'au moins environ 10 Km.

8. Procédé selon la revendication 7, dans lequel ledit premier seuil prédéterminé est égal à 2 ou 3.

9. Procédé selon la revendication 8 lorsqu'elle dépend de la revendication 3, dans lequel ledit deuxième seuil prédéterminé est égal à 0.
